# EUROPEAN PATENT APPLICATION

(11) **EP 4 563 173 A1**
(43) Date of publication of application: **04.06.2025**
(21) Application number: 23860623.0
(22) Date of filing: 26.06.2023
(51) Int. Cl.: A61M 5/145, A61M 5/172, A61M 5/142, G16H 20/17, G16H 50/20, G16H 40/63

(54) **DRUG INJECTION DEVICE AND METHOD**

(30) Priority: 29.08.2022 KR 20220108106; 07.06.2023 KR 20230072560
(71) Applicant: Caremedi Co., Ltd., Seoul 07207 (KR); Sogang University Research & Business Development Foundation, Seoul 04107 (KR)
(72) Inventor: SHIN, Woonsup, Seoul 04065 (KR); ZHU, Enhua, Seoul 04109 (KR); SHIN, Yeong Jong, Gimpo-si Gyeonggi-do 10126 (KR)
(74) Representative: Manitz Finsterwald Patent- und Rechtsanwaltspartnerschaft mbB
(86) International application number: PCT/KR2023/008824
(87) International publication number: WO 2024/048939

(57) **Abstract**

A drug injection device includes a drug injector configured to suck a drug from a drug storage by electrochemically operating an electroosmotic pump and discharge an sucked drug to an injection target; and a controller configured to output, to the drug injector, a control signal corresponding to an amount-based injection sequence for causing the electroosmotic pump to operate in an immediate bolus injection mode, wherein the amount-based injection sequence includes at least one pulse block defining a voltage pulse or a current pulse to be applied to the electroosmotic pump, the at least one pulse block defines a pair of pulse signals including a forward pulse and a reverse pulse that are applied to the electroosmotic pump to alternately generate negative pressure and positive pressure, and the electroosmotic pump alternately generates negative pressure and positive pressure for each pulse block to suck and discharge the drug.

## Description

### BACKGROUND

The present invention relates to a drug injection device and method.

Drugs may be injected into the body in various ways, such as oral, subcutaneous, and intravenous administration, depending on the type, purpose of treatment, and method. A drug injection device using a drug pump may automatically inject drugs into the body at a desired speed and dose at a required time. Therefore, a drug injection device using a drug pump may be used not only in hospitals or in patients' daily living environments, but also in various forms.

An insulin pump, which is commonly called an insulin injector, is a medical device that supplies insulin into the body from the outside at a set time to accurately control blood sugar levels, similar to a pancreas, for diabetic patients who do not secrete insulin or secrete only a small amount of insulin.

Such an insulin pump is used by insulin-dependent diabetic patients, and may continuously inject drugs for 24 hours in a state of being attached to the patient. In this way, an insulin injector has to periodically inject drugs for a long period of time for diabetic patients, and accordingly, active technology development is being conducted to miniaturize and automate insulin injectors for the convenience of users.

However, as insulin injectors are miniaturized and automated, there are cases where it is difficult to actually secure the stability of the operation of the insulin injector, and this is mostly due to the back pressure caused by various reactions between the drug and the biological fluid at the tip of the cannula or needle, which is the main cause of the blockage phenomenon. In particular, when the amount of injected drug is small or is injected slowly, the blockage phenomenon becomes more serious.

Therefore, a method of continuously inject drugs into patients, stably inject an accurate amount of drugs, and control the injection of drugs such that a path is not blocked during the drug injection is required.

The present invention provides a drug injection method for immediate bolus injection by a drug injection device based on an electroosmotic pump.

However, technical issues to be solved by the present embodiments are not limited to the technical issues described above, and other technical issues may be derived therefrom.

### SUMMARY

A drug injection device according to an embodiment of the present invention includes a drug injector configured to suck a drug from a drug storage by electrochemically operating an electroosmotic pump and discharge an sucked drug to an injection target; and a controller configured to output, to the drug injector, a control signal corresponding to an amount-based injection sequence for causing the electroosmotic pump to operate in an immediate bolus injection mode, wherein the amount-based injection sequence includes at least one pulse block defining a voltage pulse or a current pulse to be applied to the electroosmotic pump, the at least one pulse block defines a pair of pulse signals including a forward pulse and a reverse pulse that are applied to the electroosmotic pump to alternately generate negative pressure and positive pressure, and the electroosmotic pump alternately generates negative pressure and positive pressure for each pulse block to suck and discharge the drug.

A drug injection method according to an embodiment of the present invention includes setting an amount-based injection sequence for causing the drug injection device to operate in an immediate bolus injection mode; applying a control signal corresponding to the amount-based injection sequence to the electroosmotic pump; and causing the electroosmotic pump to alternately generate negative pressure and positive pressure for each pulse block in response to the control signal such that the drug is sucked and discharged, wherein the amount-based injection sequence includes at least one pulse block defining one of a voltage pulse and a current pulse applied to the electroosmotic pump, and the at least one pulse block defines a pair of pulse signals including a forward pulse and a reverse pulse that are applied to the electroosmotic pump to alternately generate the negative pressure and the positive pressure.

According to the present invention described above, a drug injector using an electroosmotic pump may inject a fixed amount of drug by setting an amount-based injection sequence according to drug injection conditions.

### BRIEF DESCRIPTION OF THE DRAWINGS

FIG. 1 is a block diagram schematically illustrating a drug injection device according to an embodiment of the present invention.
FIG. 2 is a block diagram schematically illustrating a configuration of a drug injector illustrated in FIG. 1.
FIG. 3 is a conceptual diagram schematically illustrating an amount-based injection sequence according to an embodiment of the present invention.
FIG. 4 is a table showing an example of multiple pulse blocks according to an embodiment of the present invention.
FIG. 5 is an example diagram illustrating a signal structure for an amount-based injection sequence according to an embodiment of the present invention.
FIG. 6 is an example diagram illustrating a signal structure for a speed-based injection sequence according to an embodiment of the present invention.
FIG. 7 is a table showing an example of a basal injection mode according to an embodiment of the present invention.
FIG. 8 is an example diagram illustrating a signal structure for the basal injection mode illustrated in FIG. 7.
FIG. 9 is an example diagram illustrating a signal structure in which an amount-based injection sequence is applied in the basal injection mode illustrated in FIG. 8.
FIG. 10 is a block diagram schematically illustrating a configuration of the electroosmotic pump illustrated in FIG. 2.
FIG. 11 is a block diagram schematically illustrating a configuration of a driver illustrated in FIG. 10.
FIG. 12 is an example diagram schematically illustrating a configuration of a driver illustrated in FIG. 6.
FIG. 13 is an application example diagram of a drug injection device according to an embodiment of the present invention.
FIG. 14 is a block diagram schematically illustrating a configuration of the drug injection device illustrated in FIG. 13.
FIG. 15 is a block diagram schematically illustrating a configuration of the drug injection device illustrated in FIG. 13.
FIG. 16 is a flowchart illustrating a drug injection method according to an embodiment of the present invention.
FIG. 17 is a flowchart illustrating a process of applying a control signal illustrated in FIG. 16 to an electroosmotic pump.

### DETAILED DESCRIPTION OF THE EMBODIMENTS

Hereafter, the present disclosure is described in detail with reference to the accompanying drawings. However, the present disclosure may be implemented in many different forms and is not limited to the embodiments described herein. In addition, the accompanying drawings are only for easy understanding of the embodiments disclosed in the present specification, and the technical ideas disclosed in the present specification are not limited by the accompanying drawings. In order to clearly describe the present disclosure in the drawings, parts irrelevant to the descriptions are omitted, and a size, a shape, and a form of each component illustrated in the drawings may be variously modified. The same or similar reference numerals are assigned to the same or similar portions throughout the specification.

Suffixes "module" and "unit" for the components used in the following description are given or used interchangeably in consideration of ease of writing the specification, and do not have meanings or roles that are distinguished from each other by themselves. In addition, in describing the embodiments disclosed in the present specification, when it is determined that a detailed descriptions of related known technologies may obscure the gist of the embodiments disclosed in the present specification, the detailed descriptions are omitted.

Throughout the specification, when a portion is said to be "connected (coupled, in contact with, or combined)" with another portion, this includes not only a case where it is "directly connected (coupled, in contact with, or combined)" ", but also a case where there is another member therebetween. In addition, when a portion "includes (comprises or provides)" a certain component, this does not exclude other components, and means to "include (comprise or provide)" other components unless otherwise described.

Terms indicating ordinal numbers, such as first and second, used in the present specification are used only for the purpose of distinguishing one component from another component and do not limit the order or relationship of the components. For example, the first component of the present disclosure may be referred to as the second component, and similarly, the second component may also be referred to as the first component.

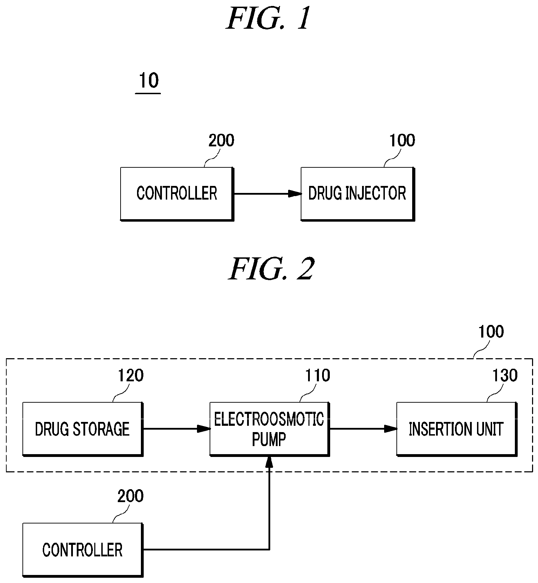

FIG. 1 is a block diagram schematically illustrating a configuration of a drug injection device according to an embodiment of the present invention, and FIG. 2 is a block diagram schematically illustrating a configuration of the drug injection device illustrated in FIG. 1.

Referring to FIG. 1 and FIG. 2, a drug injection device 10 according to an embodiment of the present invention includes a drug injector 100 and a controller 200.

The drug injector 100 sucks a drug from a drug storage by electrochemically operating an electroosmotic pump 110 in an immediate bolus injection mode and discharges the sucked drug to an injection target. In addition, the controller 200 outputs a control signal corresponding to an amount-based injection sequence for causing the drug injector 100 to operate the electroosmotic pump 110 in the immediate bolus injection mode. Here, the immediate bolus injection mode is a drug injection mode in which a predetermined amount of drug is immediately injected into a user to lower a blood sugar level to a normal range when the user's blood sugar level has risen sharply or is expected to rise.

The controller 200 may mean a data processing device which is embedded in hardware and includes a physically structured circuit to perform a function expressed as a code or command included in a program. The data processing device embedded in hardware may include, for example, a microprocessor, a central processing unit (CPU), a processor core, a multiprocessor, an application-specific integrated circuit (ASIC), a field programmable gate array (FPGA), a micro control unit (MCU), an embedded processor, or so on, but the scope of the present invention is not limited thereto.

Meanwhile, the controller 200 may be embedded solely in the drug injection device 10, or may control the immediate bolus injection mode of the drug injection device 10 by being connected to a user terminal 40 through a communication module and being integrated into the user terminal 40.

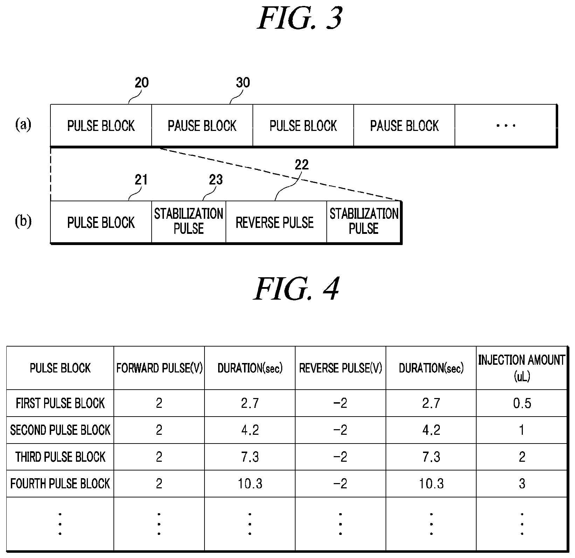

FIG. 3 is a diagram illustrating a concept of the amount-based injection sequence according to an embodiment of the present invention, and the amount-based injection sequence is specifically described with reference to FIG. 3.

The amount-based injection sequence includes multiple pulse blocks 20 that satisfy a drug injection amount according to a drug injection condition including the drug injection amount. In this case, the immediate bolus injection sequence has a configuration in which the multiple pulse blocks 20 that satisfy the drug injection amount are arranged continuously without any gap or any pause time. The immediate bolus injection is defined as a bolus injection in a general insulin injection device.

Referring to FIG. 3, the amount-based injection sequence includes the multiple pulse blocks 20 continuously arranged without any pause time. Each of the multiple pulse blocks 20 defines a voltage pulse or current pulse to be applied to the electroosmotic pump 110, and the amount of drug to be injected changes depending on durations.

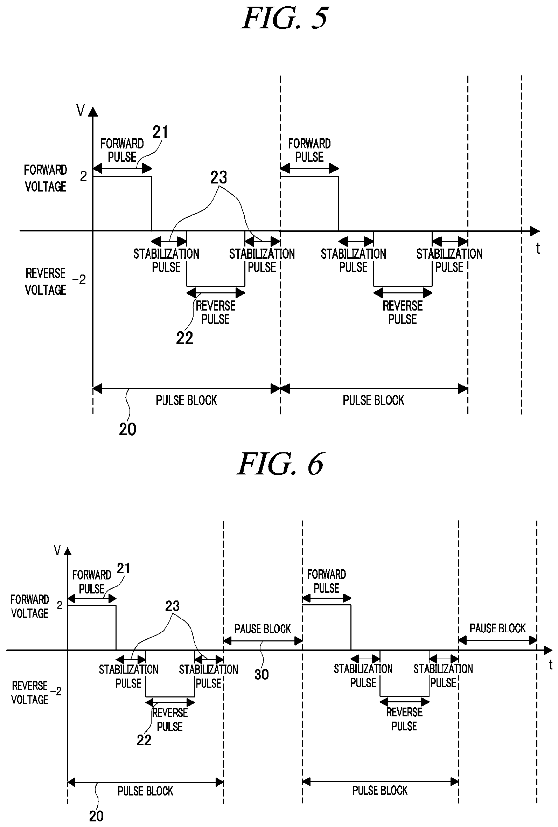

FIG. 4 is a table showing an example of multiple pulse blocks according to an embodiment of the present invention, and FIG. 5 is an example diagram illustrating a signal structure of an amount-based injection sequence according to an embodiment of the present invention.

Referring to FIG. 4 and FIG. 5, a structure of the amount-based injection sequence is set as follows to satisfy a drug injection condition including a drug injection amount. The amount-based injection sequence has a structure in which M (M is a natural number less than or equal to N that is a natural number greater than or equal to 1) pulse blocks 20 selected according to the drug injection amount among N pulse blocks 20 supplying different amounts of drugs as illustrated in FIG. 4 are sequentially arranged. Here, the minimum number of pulse blocks 20 that may satisfy the drug injection amount may be used among the multiple pulse blocks 20 that constitute the amount-based injection sequence, and the pulse block 20 that supplies the largest amount of drug among the pulse blocks 20 that may satisfy the drug injection amount is preferentially arranged.

For example, when the drug injection amount is 5 µL, the amount-based injection sequence includes a fourth pulse block that supplies 3 µL and a third pulse block that supplies 2 µL, and the fourth pulse block is arranged first and then the third pulse block is arranged. In addition, the fourth pulse block is completed without a gap, the third pulse block is executed immediately.

Next, referring to FIG. 4 and FIG. 5, each of the pulse blocks 20 includes information on a pair of pulse signals including a forward pulse and a reverse pulse, and includes information on an amplitude of each pulse and a duration for which a pulse is maintained. The pair of pulse signals including the forward pulse and the reverse pulse are applied to the electroosmotic pump 110 to alternately generate negative pressure and positive pressure, and thereby, the negative pressure and positive pressure are alternately generated in each of the pulse blocks 20 to suck and discharge a drug.

A pair of pulse signals 21 and 22 included in the pulse block 20 is a voltage pulse signal or a current pulse signal. A pair of voltage pulse signals may include a forward voltage pulse and a reverse voltage pulse, and a pair of current pulse signals may include a forward current pulse and a reverse current pulse. Here, the pair of voltage pulse signals may include information on amplitudes and durations of the respective voltage pulses, and the pair of current pulse signals may include information on amplitudes and durations of the respective current pulses. For example, in the amount-based injection sequence illustrated in FIG. 5, the amplitude of a pulse signal may be 2 volts, and the duration thereof may be 10 seconds.

Pulse blocks 20 included in the amount-based injection sequence may each include voltage pulses having different voltage levels or current pulses having different current levels. Alternatively, the pulse blocks 20 may each include pulses having the same voltage level or current pulses having the same current level, but respective durations may be set differently.

In addition, the forward pulse 21 and the reverse pulse 22 included in the pulse block 20 may be set to have the same amplitude and duration, and accordingly, amounts of drugs sucked and discharged by the electroosmotic pump 110 may be maintained to be the same.

In addition, the pair of pulse signals 21 and 22 included in the pulse block 20 may be provided as a constant voltage or a constant current, and the amount of drugs sucked and discharged by each pulse block may be adjusted by controlling the duration of a pulse signal provided by the constant voltage or the constant current. For example, the pulse blocks 20 of FIG. 5 are provided by a constant voltage of 2 volts.

Additionally, the pair of pulse signals 21 and 22 may be controlled in both amplitudes and durations, and accordingly, the amounts of drugs sucked and discharged may be adjusted to be the same. For example, an amplitude of the forward voltage pulse is 2 volts, the duration is 10 seconds, and the amplitude of a subsequent reverse voltage pulse is set to 1 volt, and the duration thereof is 20 seconds, and accordingly, an area of the forward pulse and an area of the reverse pulse are set to equal to each other to maintain the amount of sucked drug and the amount of discharged drug to be equal to each other.

In addition, the pulse block 20 may further include a stabilization pulse 23, and the stabilization pulse 23 is a pulse that maintains a voltage of 0 volt for a predetermined time after the forward voltage pulse 21 and the reverse voltage pulse 22 are applied. An operation of the electroosmotic pump 110 may be stabilized by the stabilization pulse 23. Here, when the pulse block 20 of the amount-based injection sequence includes a pair of current pulses, the pulse block 20 may further include the stabilization pulse 23 that maintains a current of 0 ampere for a predetermined time after the forward current pulse and the reverse current pulse are applied.

The controller 200 generates a control signal including a voltage pulse or a current pulse corresponding to the amount-based injection sequence based on information of the pulse block 20 included in the amount-based injection sequence and applies the control signal to the drug injector 100. The drug injector 100 that receives the control signal immediately performs the immediate bolus injection mode through the amount-based injection sequence, and when the drug injector 100 operates in the basal injection mode by the preset speed-based injection sequence, the controller 200 pauses an output of the control signal corresponding to the speed-based injection sequence and outputs the control signal corresponding to the amount-based injection sequence to the drug injector 100.

Thereafter, when the drug injector 100 completes the immediate bolus injection mode, the controller 200 outputs the control signal for the paused speed-based injection sequence to the drug injector 100 again. Here, the controller 200 omits the operation set for the speed-based injection sequence while the immediate bolus injection mode is performed, and outputs the control signal for the speed-based injection sequence set at a point in time when the operation of the immediate bolus injection mode is completed.

FIG. 6 is an example diagram illustrating a signal structure of a speed-based injection sequence according to an embodiment of the present invention. Referring to FIG. 6, the speed-based injection sequence includes at least one pulse block 20 defining a voltage pulse or current pulse applied to the electroosmotic pump 110 and at least one pause block 30 that maintains a voltage of 0 volt or a current of 0 ampere for a predetermined time after the at least one pulse block is applied.

In addition, the basal injection mode includes multiple speed-based injection sequences arranged sequentially during a set drug injection period. The basal injection mode may divide a drug injection period into multiple sequences according to a user's setting. Here, each segment includes information on a duration and a drug injection speed during the duration, and each segment is configured by repeatedly arranging the set speed-based injection sequence for a unit time to satisfy the drug injection speed during the duration. Here, the duration and drug injection speed of each segment may be set by a user.

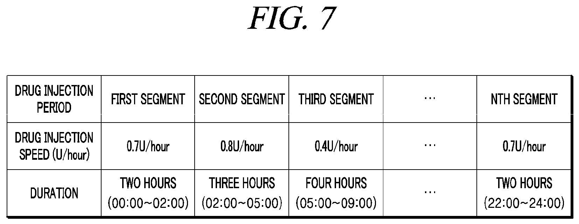

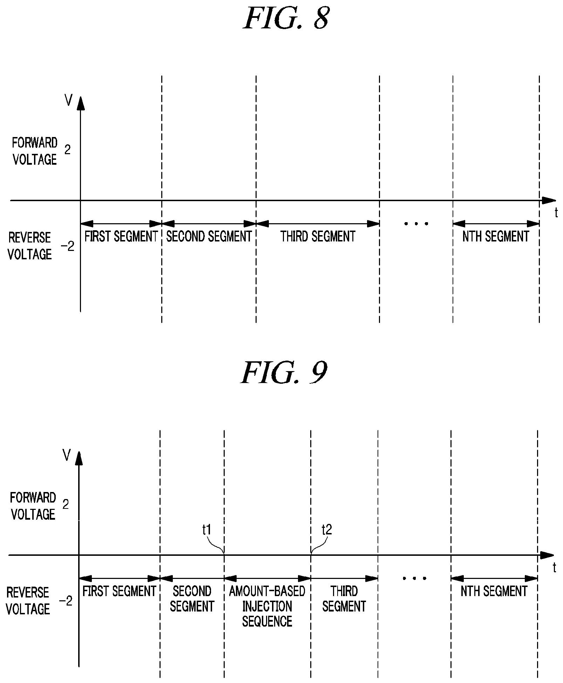

FIG. 7 is a table showing an example of a basal injection mode according to an embodiment of the present invention, FIG. 8 is an example diagram illustrating a signal structure of the basal injection mode illustrated in FIG. 7, and FIG. 9 is an example diagram illustrating a signal structure in which an amount-based injection sequence is applied to the basal injection mode illustrated in FIG. 8.

Referring to FIG. 7 and FIG. 8, in the basal injection mode illustrated in FIG. 7, 24 hours are divided into multiple segments, and a duration and a drug injection speed are set for each segment. The drug injector 100 performs the basal injection mode by performing each segment in the order illustrated in FIG. 8. When the injection mode is immediately executed at time t1 while the drug injector 100 is performing the second segment, an amount-based injection sequence is arranged in the middle of the second segment as illustrated in FIG. 9, and the amount-based injection sequence is performed. In addition, when the immediate bolus injection mode is terminated at time t2, the controller 200 transmits the speed-based injection sequence set at the time t2 to the drug injector 100, and the drug injector 100 performs the basal injection mode again. Here, a section between the time t1 of the speed-based injection sequence and the time t2 of the speed-based injection sequence is omitted due to the amount-based injection sequence.

When a control signal corresponding to the amount-based injection sequence is applied to the electroosmotic pump 110, the electroosmotic pump 110 alternately generates negative pressure and positive pressure for each pulse block to suck and discharge drugs. Here, the control signal may be a signal including a voltage pulse pair including a forward voltage pulse and a reverse voltage pulse in units of the pulse block 20 or a current pulse pair including a forward current pulse and a reverse current pulse in units of pulse blocks.

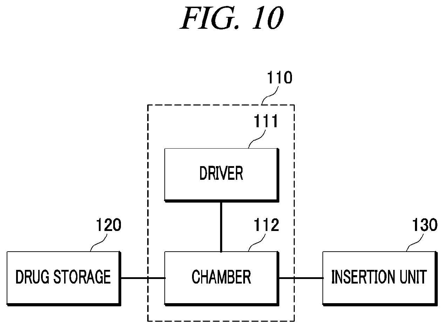

FIG. 10 is a block diagram schematically illustrating a configuration of the electroosmotic pump 110 illustrated in FIG. 2.

Referring to FIG. 10, the electroosmotic pump 110 includes a driver 111 and a chamber 112. The driver 111 is electrochemically driven by a control signal to generate positive pressure and negative pressure and discharges a drug according to the positive pressure and negative pressure, and the chamber 112 sucks a drug from a drug storage 120 according to the pressure of the driver 111 and then discharges the drug to an insertion unit 130. Here, the drug is a drug that has to be injected into a certain patient and may be, for example, insulin that is injected into a diabetic patient.

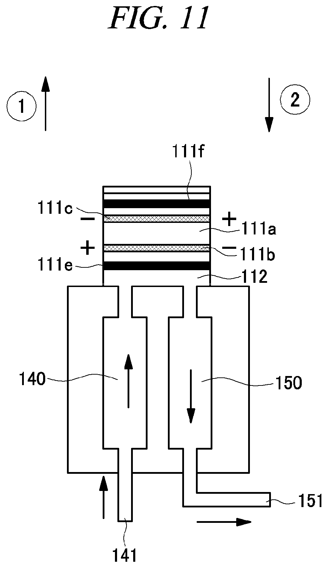

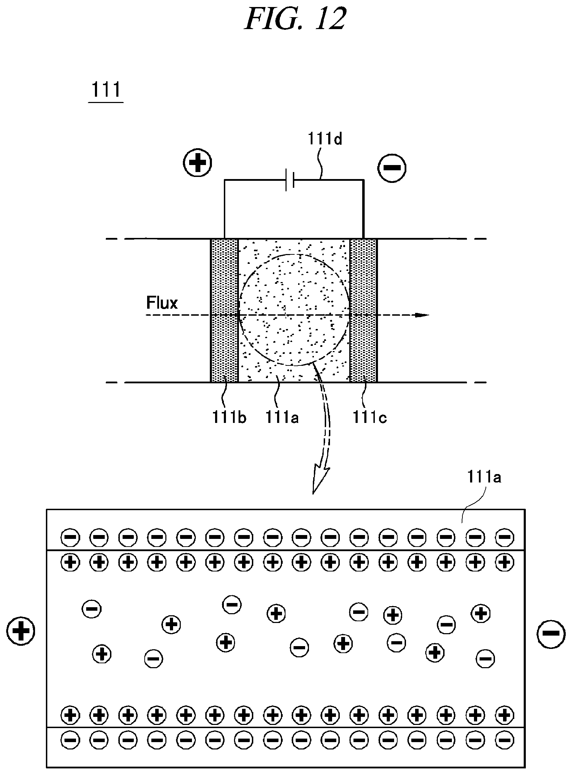

FIG. 11 is a block diagram schematically illustrating a configuration of the driver 111 illustrated in FIG. 10, and FIG. 12 is an example diagram schematically illustrating a configuration of the driver 111 illustrated in FIG. 11.

Referring to FIG. 9 and FIG. 10, the driver 111 is a pump that uses the movement of fluid according to an electroosmotic phenomenon that occurs when a voltage or current is applied by using electrodes at both ends of a porous film (membrane) 111a. The driver 111 may include the membrane 111a, a power source 111d that applies a voltage or current to a first electrode 111b and a second electrode 111c arranged on both sides of the membrane 111a, and a flow path through which a fluid moves.

In addition, the driver 111 may include a first diaphragm 111e arranged adjacent to the first electrode 111b and a second diaphragm 111f arranged adjacent to the second electrode 111c. The first and second diaphragms 111e and 111f are provided respectively one side and the other side of the membrane 111a, and shapes of the first and second diaphragms 111e and 111f are deformed by the movement of a pumping solution as positive pressure and negative pressure are alternately generated. For example, the first diaphragm 111e and the second diaphragm 111f transfer the negative pressure and positive pressure generated by an operation of the membrane 111a to a transfer target fluid. More specifically, when negative pressure is generated, at least a part of the first diaphragm 111e and the second diaphragm 111f moves backward (moving in a direction ①) such that the transfer target fluid is sucked to the chamber 112, and in contrast to this, when positive pressure is generated, at least a part of the first diaphragm 111e and the second diaphragm 111f moves forward (moving in a direction ②) such that the transfer target fluid is discharged from the chamber 112.

Silica, glass, and so on are generally used as a material of the porous film 111a, and when the silica and glass are immersed in an aqueous solution, surfaces thereof are negatively charged. There are many paths, through which fluids may pass, in the porous film 111a, and when one of the paths is enlarged, a surface of the fluid path with negative charges (bound anions) may be in a pseudostate in which the negative charges are balanced by mobile cations having positive charges. In this state, when a positive voltage is applied to the first electrode 111b and a negative voltage is applied to the second electrode 111c, negative pressure is generated, and the fluid inside the driver 111 moves in the direction ① due to the negative pressure. In this case, a drug is sucked through an inflow path 141 and flows into the chamber 112 through an inflow valve 140. In this case, an outflow valve 150 is closed such that the negative pressure is not transferred to an outflow path 151. In contrast to this, when a negative voltage is applied to the first electrode 111b and a positive voltage is applied to the second electrode 111c, the positive pressure in an opposite direction is generated by a reversible electrochemical reaction. The fluid inside the driver 111 moves in the direction ② by the positive pressure. In this case, the drug stored in the chamber 112 is introduced into a target through the outflow valve 150 and the outflow path 151. In this case, the inflow valve 140 is blocked such that the positive pressure is not transferred to the inflow path 141.

This phenomenon is called an electroosmotic phenomenon, and a pump that uses the principle is the electroosmotic pump 110.

An electrode used for the driver 111 may be implemented by a platinum mesh such as a porous electrode, porous carbon paper or fiber, or various electrode materials applied onto a porous structure so as to smoothly move a fluid.

In addition, the electrode used for the driver 111 may be coated with various fixable materials, such as an impermeable substrate material, by drop coating or spin coating. In this case, the impermeable substrate material is a plate-shaped substrate including at least one of a conductive material, a semiconductor material, and a non-conductive material, and an electrode material applied thereon may include metal, metal oxide, conducting polymer, metal hexacyanoferrate, a carbon nanostructure, or a composite thereof.

The driver 111 alternately supplies polarities of voltages or polarities of currents to the first electrode 111b and the second electrode 111c, and thereby, an electrochemical reaction occurs reversibly in both forward and reverse directions. As the electrochemical reaction occurs repeatedly in the forward and reverse directions, a fluid inside the electroosmotic pump repeatedly performs a reciprocating motion. In addition, the fluid in the first electrode 111b and the second electrode 111c is consumed and regenerated repeatedly by the reversible electrochemical reaction in the repeated forward and reverse directions, When the inflow valve 140 and the outflow valve 150 are coupled to the chamber 112 of the electroosmotic pump 110, the drug in the drug storage 120 is sucked through the inflow path 141 during an inflow operation and stored in the chamber 112 through the inflow valve 140. In addition, during an outflow operation, the drug stored in the chamber 112 is discharged to the insertion unit 130 through the outflow valve 150 and the outflow path 151.

Therefore, the pressure generated by the electroosmotic pump 110 and the volume of a discharged drug may be adjusted by controlling the magnitude and application time of a voltage or a current applied to the first and second electrodes 111b and 111c.

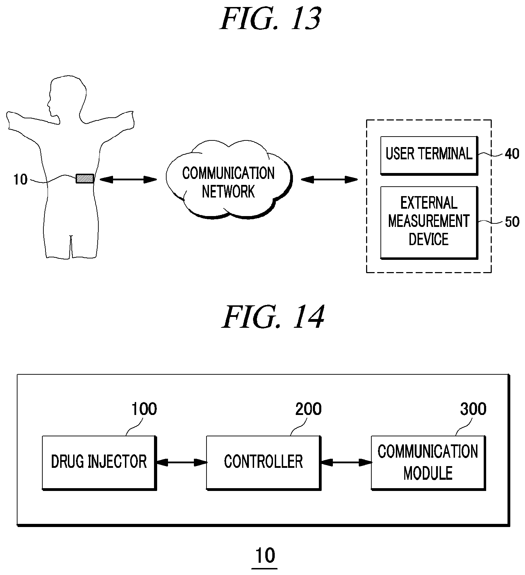

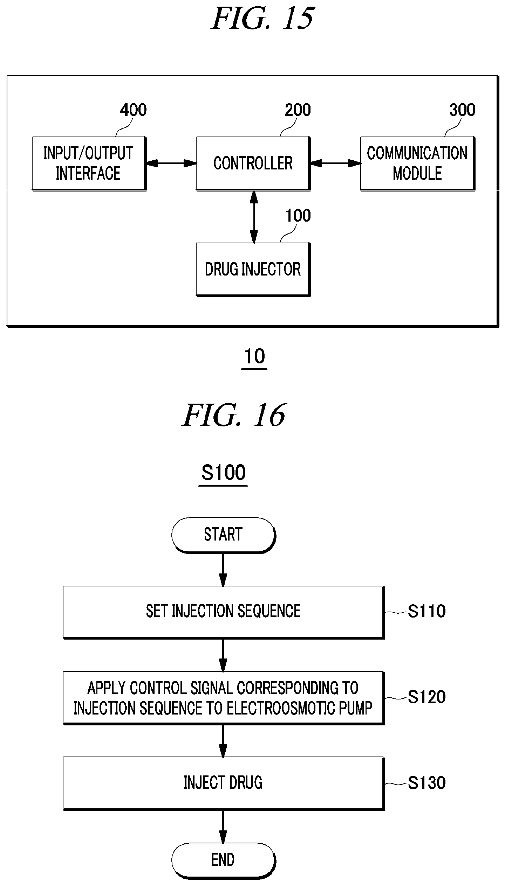

FIG. 13 is an application example diagram of a drug injection device according to an embodiment of the present invention, FIG. 14 is a block diagram schematically illustrating a configuration of the drug injection device illustrated in FIG. 13, and FIG. 15 is a block diagram schematically illustrating the configuration of the drug injection device illustrated in FIG. 13. An operation of the drug injection device 10 is specifically described with reference to FIG. 13 to FIG. 15.

The drug injection device 10 receives predetermined information from a user and sets an amount-based injection sequence based on the information, and a process of setting the amount-based injection sequence may be performed according to data used to set the amount-based injection sequence. Hereinafter, the process of setting the amount-based injection sequence is described.

The process of setting, by the drug injection device 10, the amount-based injection sequence according to an embodiment of the present invention is described with reference to FIG. 14.

The drug injection device 10 may set the amount-based injection sequence by using a drug injection condition. Here, the drug injection condition includes a drug injection amount. The drug injection device 10 may further include a communication module 300 to receive a drug injection condition from the user terminal 40. The drug injection condition is received from a user through the user terminal 40 that is communicably connected to the drug injection device 10 through the communication module 300. Then, the controller 200 sets an amount-based injection sequence corresponding to the drug injection condition and transmits a control signal corresponding the amount-based injection sequence to the drug injector 100.

Here, the amount-based injection sequence is not set by using the drug injection condition but may be directly selected by a user through the user terminal 40 or may be received by being calculated by an external computing device based on the user's past use history of the drug injection device.

The user terminal 40 may include a computer or portable terminal that may be connected to the drug injection device 10 through wireless communication. Here, the computer includes, for example, a desktop computer, a laptop computer, or so on in which a web browser is stored, and the portable terminal is, for example, a wireless communication device that ensures portability and mobility and may include all kinds of handheld-based wireless communication devices, such as various smartphones, tablet personal computers (PCs), and smart watches. The user terminal 40 is managed by a wearer of the drug injection device 10 or a medical professional, and may set a drug injection condition through an application running on the user terminal 40.

In addition, the drug injection device 10 may set the amount-based injection sequence by using user input data. Here, the user input data includes a user's blood sugar level or the user's meal information. The user input data is received from a user through the user terminal 40 connected to the drug injection device 10 through the communication module 300, and the controller 200 calculates the drug injection condition based on the received user input data. In addition, the controller 200 sets an amount-based injection sequence corresponding to the drug injection condition, and transmits a control signal corresponding to the amount-based injection sequence to the drug injector 100.

In addition, the drug injection device 10 may set the amount-based injection sequence by using user's biometric measurement data. Here, the user's biometric measurement data includes a user's blood sugar information. The user's biometric measurement data is received through an external measurement device 50 connected to the drug injection device 10 through the communication module 300, and the controller 200 calculates a drug injection condition based on the received user's biometric measurement data. In addition, an amount-based injection sequence corresponding to the drug injection condition is set, and a control signal corresponding to the amount-based injection sequence is transmitted to the drug injector 100. Here, the external measurement device 50 may be a device that senses a user's blood sugar level.

Next, a process of setting an amount-based injection sequence by the drug injection device is described with reference to FIG. 15.

The drug injection device 10 may set the amount-based injection sequence by using a drug injection condition. The drug injection device 10 may further include a user input/output interface module 400, and receives a drug injection condition from a user through the user input/output interface module 400. In an embodiment in which the drug injection device 10 includes the user input/output interface module 400, a user may control an operation of the drug injection device 10 by directly inputting a drug injection condition and so on to the user input/output interface module 400 without separately using the user terminal 40. In this case, the communication module 300 may be optionally excluded.

The controller 200 sets an amount-based injection sequence corresponding to the drug injection condition input through the user input/output interface module 400 and transmits a control signal corresponding to the amount-based injection sequence to the drug injector 100. Here, the amount-based injection sequence may be directly selected by a user through the user input/output interface module 400 without being set by using the drug injection condition.

The user input/output interface module 400 may include a physical input/output button coupled to an external housing including the drug injection device 10, and a signal processing circuit that transmits a signal generated according to an operation of the physical input/output button to the controller 200.

Alternatively, the user input/output interface module 400 may output a user interface (UI) that guides a user to input information on a drug injection condition or amount-based injection sequence through a display including a touch screen.

Additionally, in the process of setting the amount-based injection sequence described above, the controller 200 may also set an amount-based injection sequence corresponding to each drug injection condition by referring to a table in which multiple amount-based injection sequences are stored for each drug injection condition.

FIG. 16 is a flowchart illustrating a drug injection method according to an embodiment of the present invention.

Referring to FIG. 1, FIG. 2, and FIG. 16, a drug injection method S100 according to an embodiment of the present invention includes step S110 of setting an amount-based injection sequence for causing the drug injection device 10 to operate in an immediate bolus injection mode, and step S120 of applying a control signal corresponding to the amount-based injection sequence to the electroosmotic pump 110. Thereafter, in response to the control signal, the electroosmotic pump 110 alternately generates negative pressure and positive pressure for each pulse block to suck and discharge a drug and inject the drug into a user (step S130). Here, the amount-based injection sequence includes at least one pulse block defining a voltage pulse or current pulse applied to the electroosmotic pump 110, and each pulse block defines a pair of pulse signals including a forward pulse and a reverse pulse that are applied to the electroosmotic pump 110 to alternately generate the negative pressure and positive pressures.

Hereinafter, each step is described in detail.

In the process (step S110) of setting the amount-based injection sequence, the drug injection device (10) may set the amount-based injection sequence by using a drug injection condition, and may have various embodiments depending on when the drug injection condition is input. Here, the drug injection condition includes a drug injection amount.

The drug injection device 10 may receive the drug injection condition from a user through the user terminal 40 connected to the drug injection device 10 through the communication module 300. Then, the controller 200 sets an amount-based injection sequence corresponding to the drug injection condition and transmits a control signal corresponding the amount-based injection sequence to the drug injector 100. Here, the amount-based injection sequence may be directly selected by a user through the user terminal 40 without being set by using the drug injection condition.

In addition, the drug injection device 10 may receive the drug injection condition from the user through the user input/output interface module 400, and the controller 200 sets an amount-based injection sequence corresponding to the drug injection condition and transmits a control signal to the drug injector 100. Here, the amount-based injection sequence may be directly selected by a user through the user input/output interface module 400 without being set by using the drug injection condition.

In addition, the drug injection device 10 may set the amount-based injection sequence by using user input data. Here, the user input data includes a user's blood sugar level or a user's meal information. The user input data is received from a user through the user terminal 40 communicatively connected to the drug injection device 10 through the communication module 300, and the controller 200 calculates the drug injection condition based on the received user input data. In addition, the amount-based injection sequence corresponding to the drug injection condition is set, and the control signal corresponding to the amount-based injection sequence is transmitted to the drug injector 100.

In addition, the drug injection device 10 may set the amount-based injection sequence by using a user's biometric measurement data. Here, the user's biometric measurement data includes a user's blood sugar level information. The user's biometric measurement data is received through the external measurement device 50 communicatively connected to the drug injection device 10 through the communication module 300, and the controller 200 calculates the drug injection condition based on the received user's biometric measurement data. In addition, the amount-based injection sequence corresponding to the drug injection condition is set, and the control signal corresponding to the amount-based injection sequence is transmitted to the drug injector 100. Here, the external measurement device 50 may be a device that senses a user's blood sugar level.

Meanwhile, in the process of setting the amount-based injection sequence, the controller 200 may set the amount-based injection sequence corresponding to the drug injection condition by referring to a table in which multiple amount-based injection sequences are stored for each drug injection condition.

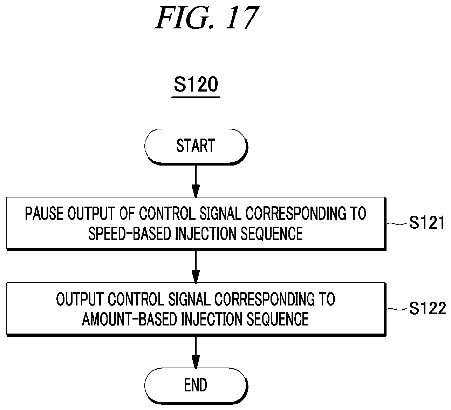

FIG. 17 is a flowchart illustrating a process of applying a control signal illustrated in FIG. 16 to an electroosmotic pump.

Referring to FIG. 17, in a process of applying a control signal to an electroosmotic pump (step S120), when the drug injector 100 operates in a basal injection mode by a preset speed-based injection sequence, the controller 200 pauses an output of a control signal corresponding to the speed-based injection sequence (step S121) and outputs a control signal corresponding to the amount-based injection sequence to the drug injector 100 (step S122).

In addition, in a process of applying a control signal to the electroosmotic pump (step S120), the control signal corresponding to an amount-based injection sequence may include a voltage pulse pair including a forward voltage pulse and a reverse voltage pulse or a current pulse pair including a forward current pulse and a reverse current pulse in pulse block units, and in response to the control signal, the electroosmotic pump 110 may perform an operation of alternately generating negative pressure and positive pressure for each pulse block to suck and discharge a drug, thereby injecting the drug into a user in an immediate bolus injection mode (step S130).

Thereafter, when the immediate bolus injection mode is completed, the controller 200 outputs a control signal for the paused speed-based injection sequence to the drug injector 100 again. Here, the controller 200 omits the operation set for the speed-based injection sequence while the immediate bolus injection mode is performed, and outputs a control signal for the speed-based injection sequence set at a point in time when the operation of the immediate bolus injection mode is completed.

A method according to an embodiment of the present disclosure may be performed in the form of a recording medium including instructions executable by a computer, such as a program module executed by a computer. A computer readable medium may be any available medium that may be accessed by a computer and includes both volatile and nonvolatile media, removable and non-removable media. Also, the computer readable medium may include a computer storage medium. A computer storage medium includes both volatile and nonvolatile media and removable and non-removable media implemented by any method or technology for storing information, such as computer readable instructions, data structures, program modules or other data.

In addition, although the method and system of the present disclosure are described with respect to specific embodiments, some or all of components or operations thereof may be implemented by using a computer system having a general-purpose hardware architecture.
those skilled in the art to which the present disclosure belongs will understand that the present disclosure may be easily modified into another specific form based on the descriptions given above without changing the technical idea or essential features of the present disclosure. Therefore, the embodiments described above should be understood as illustrative in all respects and not limiting. The scope of the present invention is indicated by the claims described below, and all changes or modified forms derived from the meaning, scope of the claims, and their equivalent concepts should be interpreted as being included in the scope of the present invention.

The scope of the present application is indicated by the claims described below rather than the detailed description above, and all changes or modified forms derived from the meaning, scope of the claims, and their equivalent concepts should be interpreted as being included in the scope of the present application.

## Claims

1. A drug injection device comprising:
a drug injector configured to suck a drug from a drug storage by electrochemically operating an electroosmotic pump and discharge an sucked drug to an injection target; and
a controller configured to output, to the drug injector, a control signal corresponding to an amount-based injection sequence for causing the electroosmotic pump to operate in an immediate bolus injection mode,
wherein the amount-based injection sequence includes at least one pulse block defining a voltage pulse or a current pulse to be applied to the electroosmotic pump,
the at least one pulse block defines a pair of pulse signals including a forward pulse and a reverse pulse that are applied to the electroosmotic pump to alternately generate negative pressure and positive pressure, and
the electroosmotic pump alternately generates negative pressure and positive pressure for each pulse block to suck and discharge the drug.

2. The drug injection device of claim 1, wherein
the amount-based injection sequence includes M pulse blocks (M is a natural number less than or equal to N) selected from among N pulse blocks (N is a natural number) that supply different amounts of drugs, and the largest number of pulse blocks that satisfy a target drug injection amount are arranged with priority.

3. The drug injection device of claim 1, wherein,
when the amount-based injection sequence includes multiple pulse blocks, the multiple pulse blocks are arranged sequentially.

4. The drug injection device of claim 1, wherein
a size and a duration of a forward pulse included in the pair of pulse signals are set to be equal to a size and a duration of a reverse pulse included in the pair of pulse signals such that a drug amount sucked by the electroosmotic pump is equal to a drug amount discharged by the electroosmotic pump.

5. The drug injection device of claim 1, wherein
the pair of pulse signals includes a pair of voltage pulses including a forward voltage pulse and a reverse voltage pulse and includes a stabilization pulse that maintains a voltage of 0 volt for a predetermined period of time after the forward voltage pulse and the reverse voltage pulse are applied, or
the pair of pulse signals includes a pair of current pulses including a forward current pulse and a reverse current pulse and includes a stabilization pulse that maintains a current of 0 ampere for a predetermined period of time after the forward current pulse and the reverse current pulse are applied.

6. The drug injection device of claim 1, wherein,
when the drug injector operates in a basal injection mode by a preset speed-based injection sequence, the controller pauses an output of a control signal corresponding to the speed-based injection sequence and outputs the control signal corresponding to the amount-based injection sequence, and
the speed-based injection sequence includes at least one pulse block defining a voltage pulse or a current pulse to be applied to the electroosmotic pump and at least one pause block maintaining a voltage of 0 volt or a current of 0 ampere for a predetermined period of time after the at least pulse block is applied.

7. The drug injection device of claim 6, wherein
when an operation of the immediate bolus injection mode according to the amount-based injection sequence is completed, the controller subsequently outputs the control signal for a paused speed-based injection sequence.

8. The drug injection device of claim 6, wherein
when an operation of the immediate bolus injection mode according to the amount-based injection sequence is completed, the controller subsequently outputs the control signal for a paused speed-based injection sequence, and
the controller omits an operation according to a speed-based injection sequence scheduled during a paused period and performs an operation according to a speed-based injection sequence scheduled at a point in time when an operation of an immediate bolus injection mode is completed.

9. The drug injection device of claim 1, wherein the electroosmotic pump includes:
a driver configured to alternately generate positive pressure and negative pressure by being electrochemically driven by the control signal; and
a chamber configured to suck the drug from a drug source according to pressure of a driver and discharge the drug to an insertion unit.

10. The drug injection device of claim 1, wherein
the drug injection device receives a drug injection condition from one of a user terminal and a user input/output interface module,
the controller outputs the control signal by using an amount-based injection sequence corresponding to the drug injection condition, and
the drug injection condition includes a drug injection amount.

11. The drug injection device of claim 8, wherein
the drug injection device receives user input data from a user terminal,
the controller calculates a drug injection condition based on user input data and outputs the control signal by using an amount-based injection sequence corresponding to the drug injection condition,
the user input data includes a blood sugar level, activity information, or meal information of a user, and
the drug injection condition includes a drug injection amount.

12. The drug injection device of claim 1, wherein
the drug injection device receives biometric data of a user from an external measurement device,
the controller calculates a drug injection condition based on the biometric data of the user and outputs the control signal by using an amount-based injection sequence corresponding to the drug injection condition,
the biometric data of the user includes blood sugar information or activity information of the user, and
the drug injection condition includes a drug injection amount.

13. The drug injection device of claims 8 to 10, wherein
the controller sets the amount-based injection sequence corresponding to the drug injection condition by referring to a table in which multiple amount-based injection sequences are stored for each drug injection condition.

14. The drug injection device of claim 1, wherein
the drug injection device receives an amount-based injection sequence set by one of a user terminal and a user input/output interface module, and
the controller outputs the control signal by using the amount-based injection sequence.

15. The drug injection device of claim 1, wherein
the amount-based injection sequence is calculated and received through an external computing device based on a user's past use history of the drug injection device.

16. A drug injection method of a drug injection device including an electroosmotic pump, the drug injection method comprising:
setting an amount-based injection sequence for causing the drug injection device to operate in an immediate bolus injection mode;
applying a control signal corresponding to the amount-based injection sequence to the electroosmotic pump; and
causing the electroosmotic pump to alternately generate negative pressure and positive pressure for each pulse block in response to the control signal such that the drug is sucked and discharged,
wherein the amount-based injection sequence includes at least one pulse block defining one of a voltage pulse and a current pulse applied to the electroosmotic pump, and
the at least one pulse block defines a pair of pulse signals including a forward pulse and a reverse pulse that are applied to the electroosmotic pump to alternately generate the negative pressure and the positive pressure.

17. The drug injection method of claim 16, wherein
in the setting of the amount-based injection sequence, M (M is a natural number less than or equal to N) pulse blocks selected from among N (N is a natural number) pulse blocks that supply different amounts of drugs are arranged, and a largest number of pulse blocks that satisfy a target drug injection amount are arranged first.

18. The drug injection method of claim 16, wherein
in the setting of the amount-based injection sequence, the amount-based injection sequence is set such that a drug amount sucked by a pair of voltage pulse signals or a pair of current pulse signals is equal to a drug amount discharged by the pair of voltage pulse signals or the pair of current pulse signals by controlling magnitude and a duration of the pair of voltage pulse signals or magnitude and a duration of the pair of current signals.

19. The drug injection method of claim 16, wherein
the pair of pulse signals includes a pair of voltage pulses including a forward voltage pulse and a reverse voltage pulse and includes a stabilization pulse that maintains a voltage of o V for a predetermined time after application of the forward voltage pulse and the reverse voltage pulse, or
includes a current pulse pair including a forward current pulse and a reverse current pulse and includes a stabilization pulse that maintains a current of 0 A for a predetermined time after application of the forward current pulse and the reverse current pulse.

20. The drug injection method of claim 16, wherein, in the setting of the amount-based injection sequence,
the drug injection device sets an amount-based injection sequence corresponding to a drug injection condition received from one of a user terminal and a user input/output interface module, and
the drug injection condition include a drug injection amount.

21. The drug injection method of claim 16, wherein, in the setting of the amount-based injection sequence,
the drug injection device calculates the drug injection condition based on user input data received from a user terminal and sets an amount-based injection sequence corresponding to the drug injection condition,
the user input data includes a blood sugar level and activity information of a user, and
the drug injection condition includes a drug injection amount.

22. The drug injection method of claim 16, wherein, in the setting of the amount-based injection sequence,
the drug injection device calculates the drug injection condition based on user's biometric measurement data received from an external measurement device and sets an amount-based injection sequence corresponding to the drug injection condition,
the biometric data of the user includes blood sugar information or activity information of the user, and
the drug injection condition includes a drug injection amount.

23. The drug injection method of claims 20 to 22, wherein,
in the setting of the amount-based injection sequence, the amount-based injection sequence corresponding to the drug injection condition is set by referring to a table in which multiple amount-based injection sequences are stored for each drug injection condition.

24. The drug injection method of claim 16, wherein,
in the setting of the amount-based injection sequence, an amount-based injection sequence set by one of a user terminal and a user input/output interface module is received and set as the amount-based injection sequence.

25. The drug injection method of claim 16, wherein,
in the setting of the amount-based injection sequence, an amount-based injection sequence is calculated from s user's past use history of the drug injection device through an external computing device and is set as the amount-based injection sequence.

26. The drug injection method of claim 16, wherein
the applying of the control signal includes:
pausing an output of a control signal corresponding to the speed-based injection sequence when the drug injector operates in a basal injection mode by a preset speed-based injection sequence; and
outputting the control signal corresponding to the amount-based injection sequence when the drug injector operates in the basal injection mode by the preset speed-based injection sequence, and
the speed-based injection sequence includes at least one pulse block defining a voltage pulse or a current pulse to be applied to the electroosmotic pump and at least one pause block maintaining a voltage of 0 volt voltage or a current of 0 ampere for a predetermined period of time after the at least one pulse block is applied.

27. The drug injection method of claim 26, further comprising:
subsequently outputting the control signal for the paused speed-based injection sequence when the immediate bolus injection mode according to the amount-based injection sequence is completed.
